(19) Europäisches Patentamt
European Patent Office
Office européen des brevets

(11) **EP 4 383 264 A1**

(12) **EUROPEAN PATENT APPLICATION**
published in accordance with Art. 153(4) EPC

(43) Date of publication:
**12.06.2024 Bulletin 2024/24**

(21) Application number: **22852594.5**

(22) Date of filing: **28.03.2022**

(51) International Patent Classification (IPC):
*G16H 10/40* (2018.01)    *G16H 50/70* (2018.01)
*A61B 5/00* (2006.01)    *A61B 5/02* (2006.01)
*A61B 5/145* (2006.01)    *G06Q 10/04* (2023.01)

(52) Cooperative Patent Classification (CPC):
**A61B 5/00; A61B 5/02; A61B 5/145; G06Q 10/04;
G16H 10/40; G16H 50/70**

(86) International application number:
**PCT/JP2022/015097**

(87) International publication number:
**WO 2023/013165 (09.02.2023 Gazette 2023/06)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **06.08.2021 JP 2021130123
15.03.2022 JP 2022040637**

(71) Applicant: **NISSIN FOODS HOLDINGS CO., LTD.
Yodogawa-ku
Osaka-shi, Osaka 532-8524 (JP)**

(72) Inventors:
• **ANDO, Noritaka
Osaka-shi, Osaka 532-8524 (JP)**
• **SHOBAKO, Naohisa
Osaka-shi, Osaka 532-8524 (JP)**

(74) Representative: **Mewburn Ellis LLP
Aurora Building
Counterslip
Bristol BS1 6BX (GB)**

(54) **yGT ESTIMATION DEVICE, yGT ESTIMATION METHOD, AND PROGRAM**

(57) Conventionally, a needle or the like needs to be inserted into skin of a subject to invasively collect blood in yGT measurement, which causes a psychological or physical burden on a subject. According to the present invention, it is possible to invasively estimate yGT based on attribute information and non-invasive biological information of a predetermined user by generating a yGT estimation model by machine learning based on attribute information, non-invasive biological information, and blood examination data acquired from a plurality of subjects in advance.

FIG. 1

## Description

Technical Field

The present invention relates to a yGT estimation device, a yGT estimation method, and a computer program.

Background Art

Conventionally, it has been common to measure yGT by collecting blood of a subject. However, in this method, a needle or the like needs to be invasively inserted into the skin of the subject, which causes a psychological or physical burden on the subject. Patent Literature 1 discloses a non-invasive optical measurement device configured to non-invasively measure gamma-glutamyl transpeptidase (y-GTP) from a measurement target object (such as urine, saliva, or sweat), but does not explicitly describe a specific $\gamma$-GTP measurement method or estimation accuracy. Furthermore, a certain amount of a measurement target object (such as urine, saliva, or sweat) needs to be collected before measurement.

Citation List

Patent Literature

Patent Literature 1
Japanese Patent Laid-Open No. 2004-226277

Non Patent Literature

Non Patent Literature 1
Psychology Research and Behavior Management 2011:4 81-86, Summary of the clinical investigations E. S. Teck Complex March, 20, 2010 Overview
Non Patent Literature 2
R. N. Chua, Y. W. Hau, C. M. Tiew and W. L. Hau, "Investigation of Attention Deficit/Hyperactivity Disorder Assessment Using Electro Interstitial Scan Based on Chronoamperometry Technique", in IEEE Access, vol. 7, pp. 144679-144690, 2019, doi: 10.1109/ACCESS.2019.2938095.
Non Patent Literature 3
Maarek A., Electro interstitial scan system: assessment of 10 years of research and development. Med Devices (Auckl). 2012;5:23-30. doi:10.2147/MDER.S29319

Summary of Invention

Technical Problem

The present invention is intended to provide a yGT estimation device, a yGT estimation method, and a computer program that are capable of estimating yGT based on BMI, blood pressure, electrocardiogram data, and biological impedance in addition to pulse wave data obtained through irradiation with a light beam of a particular wavelength to non-invasively and accurately estimate yGT.

Solution to Problem

A yGT estimation device according to the present invention includes an information acquisition unit configured to acquire attribute information and non-invasive biological information of a predetermined user, an estimation model storage unit configured to store a yGT estimation model, and an estimation processing unit configured to calculate a yGT estimated value of the predetermined user based on the attribute information and/or non-invasive biological information of the predetermined user by using the yGT estimation model.

The yGT estimation device further includes a training data storage unit configured to store a training data set and a learning processing unit configured to generate the yGT estimation model by machine learning based on the training data set.

The attribute information includes any one or a combination of age and sex, and the non-invasive biological information includes any one or a combination of BMI, blood pressure, pulse wave data, electrocardiogram data, and biological impedance.

The non-invasive biological information further includes oxygen saturation (SpO2).

The training data set includes attribute information, non-invasive biological information, and a blood-measured yGT measured value of a subject.

The coefficient of correlation between the logarithm of the yGT estimated value and the logarithm of the yGT measured value is equal to or larger than 0.6.

The estimation processing unit calculates a yGT risk estimated value in place of the yGT estimated value.

The learning processing unit provides labels indicating existence of the yGT risk to the training data set based on the blood-measured yGT measured value, and when a difference between the numbers of pieces of data with the yGT risk and data without the yGT risk among the labels is equal to or larger than a predetermined value, the learning processing unit increases the number of pieces of sample data in the training data set to reduce the difference.

The learning processing unit generates a first yGT risk estimation model and a second yGT risk estimation model by machine learning based on each of training data sets of different kinds, and the estimation processing unit calculates the yGT risk estimated value of the predetermined user by using the first yGT risk estimation model and the second yGT risk estimation model.

The yGT estimation device according to the present invention further includes a biological information estimation unit configured to estimate at least one piece or more of biological information among BMI, blood pressure, pulse wave data, electrocardiogram data, biological impedance, and oxygen saturation included in the biological information, and the information acquisition unit acquires, as biological information of the predetermined user, the biological information estimated by the biological information estimation unit.

A non-invasive yGT estimation system includes the yGT estimation device and a biological information measurement device configured to measure non-invasive biological information.

A yGT estimation method according to the present invention includes a step of storing a training data set including attribute information, non-invasive biological information, and a blood-measured yGT measured value of a subject, a step of generating a yGT estimation model by machine learning based on the training data set, and a step of calculating a yGT estimated value of a predetermined user based on attribute information and/or non-invasive biological information of the predetermined user by using the yGT estimation model.

A computer program according to the present invention causes a computer to execute a step of storing a training data set including attribute information, non-invasive biological information, and a blood-measured yGT measured value of a subject, a step of generating a yGT estimation model by machine learning based on the training data set, and a step of calculating a yGT estimated value of a predetermined user based on attribute information and/or non-invasive biological information of the predetermined user by using the yGT estimation model.

Advantageous Effects of Invention

According to the present invention, it is possible to extremely accurately estimate yGT or yGT risk by machine learning by using non-invasive biological information.

Brief Description of Drawings

[Figure 1] Figure 1 is a block diagram illustrating a schematic configuration of a yGT estimation system.
[Figure 2] Figure 2 is a diagram for description of an electroscangram (ESG).
[Figure 3] Figure 3 is a hardware configuration diagram of a yGT estimation device.
[Figure 4] Figure 4 is a flowchart illustrating an execution procedure of generation of a yGT estimation model by machine learning.
[Figure 5] Figure 5 is a flowchart illustrating an execution procedure of yGT estimation processing.
[Figure 6] Figure 6 is a flowchart illustrating an execution procedure of generation of a yGT risk estimation model by machine learning.
[Figure 7] Figure 7 illustrates a hierarchical structure of a neural network (NN) used for yGT risk estimation.
[Figure 8] Figure 8 is a flowchart illustrating the execution procedure of yGT risk estimation processing.
[Figure 9] Figure 9 illustrates an ROC_AUC curve of an estimation result in Example 2.
[Figure 10] Figure 10 illustrates an ROC_AUC curve of an estimation result in Example 3.

Description of Embodiment

An embodiment will be described below with reference to the accompanying drawings. Note that the embodiment is exemplary and the present invention is not limited to configurations described below.

<Device functions>

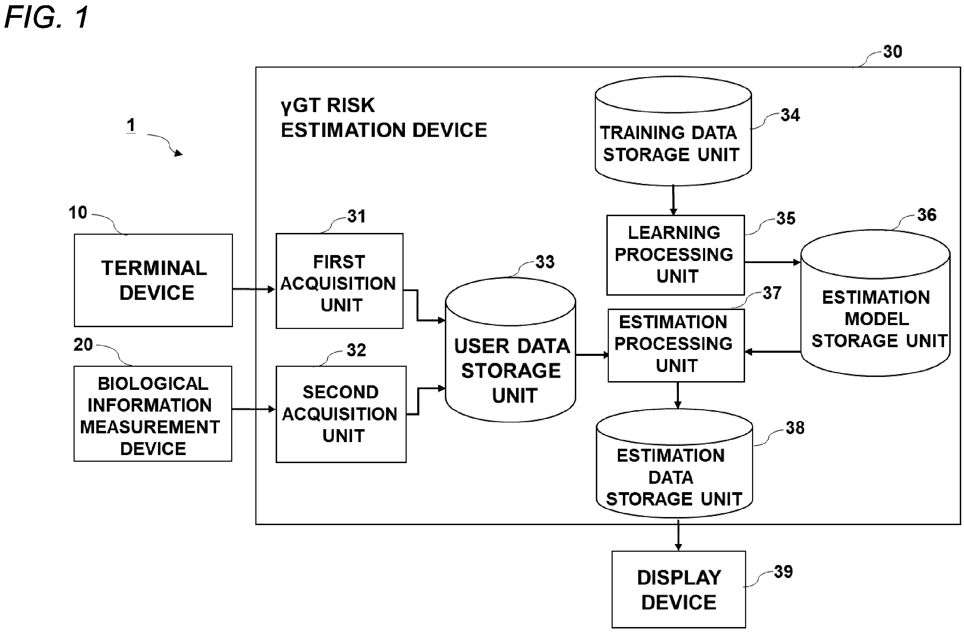

A yGT estimation system 1 and a yGT estimation device 30 according to the present embodiment will be described below with reference to Figures 1 to 8. Figure 1 is a block diagram illustrating a schematic configuration of the yGT estimation system 1 according to the present embodiment. The yGT estimation system 1 includes a terminal device 10, a biological information measurement device 20, the yGT estimation device 30, and a display device 39.

A "user" is a person who uses the yGT estimation system to non-invasively obtain an estimated value of the yGT and its risk. A "subject" is a person who provides, upon a predetermined procedure and an agreement, attribute information such as age or sex, non-invasive biological information, and a blood-measured yGT measured value as a training data set to be used in the yGT estimation system.

The terminal device 10 may be any information terminal to which the attribute information (such as full name, ID, age, or sex) of the user can be input and that can output the input information to the yGT estimation device 30 through a wired or wireless communication network. Examples of the terminal device 10 include portable terminals including a tablet terminal, a smartphone, and a wearable terminal, and include a personal computer (PC). Note that, height, weight, or the like may be measured by the biological information measurement device 20 to be described later.

The biological information measurement device 20 measures the non-invasive biological information of the user. The non-invasive biological information is biological information acquired by a method that does not require insertion of an instrument into the skin or an opening part of the body. The non-invasive biological information may be measured by using, for example, a commercially available height meter, weight meter, blood pressure meter, pulse oximeter, pulse wave meter, electrocardiogram meter, impedance measurement machine, or galvanic skin measurement machine. Alternatively, ES-TECK BC-3 (Ryobi Systems Co., Ltd.) that can simultaneously measure pulse wave data, electrocardiogram data, and biological impedance may be used. These devices can measure non-invasive biological data without causing a psychological or physical burden on the user.

In the embodiment of the present invention, the non-invasive biological information includes any one or a combination of the body mass index (BMI), blood pressure, pulse wave data, electrocardiogram data, and biological impedance and may further include oxygen saturation (SpO2).

The BMI is calculated by the following formula based on a height h [m] and a weight w [kg].

$$BMI = w/h^2 \ [kg/m^2]$$

The blood pressure includes any one or a combination of systolic blood pressure, diastolic blood pressure, pulse pressure, and average arterial blood pressure.

The pulse pressure is calculated by the following expression.

$$Pulse \ pressure = systolic \ blood \ pressure - diastolic \ blood \ pressure$$

The average arterial blood pressure is calculated by the following expression.

$$Average \ arterial \ blood \ pressure = diastolic \ blood \ pressure + pulse \ pressure \times 1/3$$

The pulse wave data is measured by irradiating, by using a sphygmograph or a pulse oximeter, a protruding body site such as a finger with red light (up to 660 nm) from a red LED and with near-infrared light (up to 905 nm) from an IR LED and measuring transmitted light thereof by using a phototransistor.

The pulse wave data includes any one or a combination of pulse, the elasticity index, the peripheral vascular resistance, the acceleration plethysmogram, b/a, e/a, -d/a, the Takazawa's second derivative of photoplethysmogram aging index, the ejection fraction, the LVET, and the dicrotic elasticity index (DEI).

The elasticity index is a numerical value obtained by dividing the height by the time from detection of a systolic peak to detection of a diastolic peak in the photoplethysmogram. The peripheral vascular resistance is calculated by "average arterial blood pressure"/"cardiac output"×80. The dicrotic elasticity index (DEI) is an indicator of the diastolic vascular elasticity and can be measured by a PWV measurement device. The DEI of 0.3 to 0.7 is normal, the DEI of 0.3 or lower indicates the possibility of high-blood pressure or arteriosclerosis, and the DEI of 0.7 or higher indicates the possibility of acute anxiety neurosis.

The acceleration plethysmogram is the second derivative of photoplethysmogram (PTG) (SDPTG). The acceleration plethysmogram is constituted by the initial positive wave (a wave), the initial negative wave (b wave), the mesosystolic re-elevation wave (c wave), the late systolic re-descent wave (d wave), and the early diastolic positive wave (e wave), and b/a, e/a, and -d/a described above are calculated from ratios of the heights of the waves. It is observed that b/a increases and c/a, d/a, and e/a decrease along with aging, and thus aging of blood vessels can be evaluated by the Takazawa's second derivative of photoplethysmogram aging index (b - c - d - e)/a. The ejection fraction is the ratio of blood transferred from the ventricles at each heartbeat and is proportional to the second derivative of photoplethysmogram aging index. The LVET stands for the left ventricular ejection time and is a time in which blood in the left ventricle is ejected to the aorta after the aortic valve is opened.

The electrocardiogram data can be measured by electrocardiography (ECG) with electrodes or by photoplethysmography (PPG).

The electrocardiogram data includes any one or a combination of the breathing rate, the heart rate, the RR interval, the standard deviation of the RR interval, the MxDMn ratio, the power spectrum in a low frequency band, the power spectrum in a high frequency band, the heart rate variation indicator LF/HF, and the total power. The RR interval is the interval from a QRS wave to the next QRS wave in the electrocardiogram. The MxDMn ratio is the ratio of the longest RR interval and the shortest RR interval in a time period and is an index of irregular heartbeats. The total power is a calculated value of the total power of the power spectrum at the frequency of 0 to 0.4 Hz (VLF, LF, HF) in a measurement of two minutes. This value reflects the entire autonomic nerve activity dominated by sympathetic nerve activity.

The high-frequency power spectrum ratio (0.1875 to 0.50 Hz; HF), the low-frequency power spectrum ratio (0.05 to 0.1875 Hz; LF), the LF/HF ratio, and the very-low-frequency power spectrum ratio (0 to 0.05 Hz; VLF) can be calculated by calculating the power spectrum density based on the electrocardiogram.

The biological impedance (conductance) can be measured by, for example, generating small current flow between two electrodes among electrodes at the six sites of the legs, the hands, and the right and left foreheads. When current flows between two of the electrodes at the six sites, (1) anode/cathode conductance ($\mu$S), (2) cathode/anode conductance ($\mu$S), (3) the difference (delta SCRA-SCRC) between the conductance measured in (1) above and the conductance measured in (2) above, and (4) electric conductivity ($\mu$S/m) can be measured. In addition, the muscle mass, the body fat amount, the total moisture content, the phase angle, and the resistance value can be simultaneously measured. In addition, the dielectric constant ($\mu$Si) can be measured for cases of conduction between the right hand and the left hand and between the right forehead and the left forehead. The biological impedance (conductance) is preferably measured by using 22 conduction patterns with the electrodes at the six sites.

The biological impedance includes any one or a combination of the body fat amount (kg), the body fat amount (%), the lean body weight, the lean body rate, the muscle mass, the total moisture content (kg), the total moisture content (%), the in-cell moisture content (%), the cardiac output, 1 forehead left-side - 2 left hand/SCR A, 1 forehead left-side - 2 left hand/delta SCR C-SCR A, 5 left hand - 6 left foot/delta SCR C-SCR A, 13 left foot - 14 right foot/SCR A, 15 right hand - 16 forehead left-side/delta SCR C-SCR A, 15 right hand -16 forehead left-side SCR C, 15 right hand - 16 forehead left-side SCRA, 19 right foot - 20 left hand/delta SCR C-SCRA, ESG 2+4+15+17 ($\mu$S/m), ESG 6+13+19 (%), ESG 6+8+19+21 (%), ESG 6+8+19+21 ($\mu$S/m), ESG 9+10 ($\mu$S/m), ESG 9+10 (%), the left-foot conductance, R ($\Omega$), the phase angle, the dielectric constant through a forehead path, forehead-path electric conductivity (9), the dielectric constant through a hand-to-hand path, hand-to-hand electric conductivity (11, 12), and the single-output amount (cardiac output/heart rate).

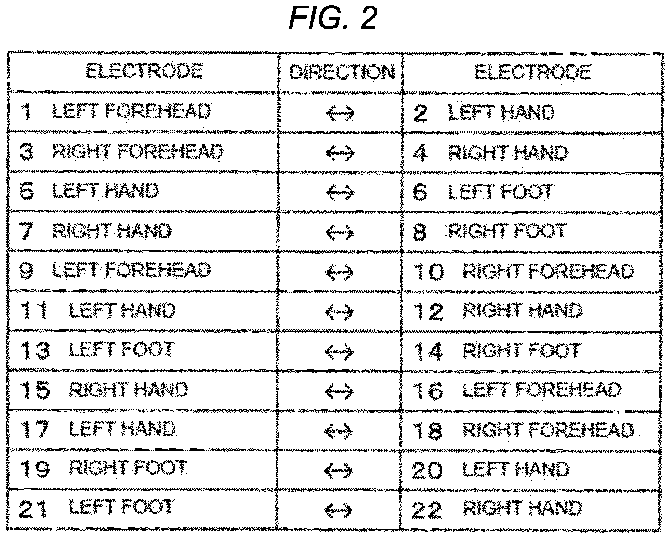

SCR stands for skin conductance response, and ESG stands for electroscangram. The symbol "+" in ESG 2+4+15+17 indicates electrodes attached to the body and used for measurement. For example, ESG 2+4+15+17 means the average of conductance values measured at the left hand when conduction is made from the left hand to the left forehead, the right hand when conduction is made from the right hand to the right forehead, the right hand when conduction is made from the right hand to the left forehead, and the left hand when conduction is made from the left hand to the right forehead as illustrated in Figure 2. Detailed description of these conductance values is provided in Non Patent Literature 1. Note that details of ESG (electroscangram) measurement methods are described in Non Patent Literatures 2 and 3.

The value "1 forehead left-side - 2 left hand/SCR A" is conductance (or conductivity) measured through a path when current flows with "1 forehead left-side" at the cathode and "2 left hand" at the anode, and the value "5 left hand - 6 left foot/delta SCR C-SCR A" is the difference between conductance values measured when conduction is made with "5 left hand" and "6 left foot" at the anode and the cathode and at the cathode and the anode.

The BMI and the blood pressure can be measured by a height-weight meter and a blood pressure meter.

The measured non-invasive biological information is output to the yGT estimation device 30 through a wired or wireless communication network. The biological information measurement device 20 may be a fixed measurement device or a portable measurement device such as a wearable terminal.

The yGT estimation device 30 includes a first acquisition unit 31, a second acquisition unit 32, a user data storage unit 33, a training data storage unit 34, a learning processing unit 35, an estimation model storage unit 36, an estimation processing unit 37, and an estimation data storage unit 38. The first acquisition unit 31 acquires the attribute information of the user from the terminal device 10. The second acquisition unit 32 acquires the non-invasive biological information of the user from the biological information measurement device 20.

The user data storage unit 33 stores the attribute information and the non-invasive biological information of the user, which are acquired from the first acquisition unit 31 and the second acquisition unit 32.

The training data storage unit 34 stores, as training data sets for machine learning, a plurality of training data sets each consisting of the attribute information and the non-invasive biological information of a plurality of subjects, which are acquired in advance and sample examination information (measured value), such as the yGT, which is obtained through a blood examination. Note that the sample examination information may further include examination information obtained from blood, urine, stool, or the like.

The learning processing unit 35 acquires the training data sets stored in the training data storage unit 34 and produces a yGT estimation model by using the training data sets. Specifically, the acquired training data sets are normalized, and the relation among the attribute information, the non-invasive biological information, and the yGT is learned by machine learning by a linear regression.

The estimation model storage unit 36 stores the yGT estimation model generated by the learning processing unit 35.

The non-invasive biological information includes any one or a combination of the BMI, the blood pressure, the pulse wave data, the electrocardiogram data, and the biological impedance. In addition, the oxygen saturation (SpO2) is included as necessary.

The estimation processing unit 37 estimates the yGT of the predetermined user based on the attribute information and/or the non-invasive biological information of the user by using the estimation model generated by the learning processing unit 35. Then, the yGT estimated value is stored in the estimation data storage unit 38.

The display device 39 can display the yGT estimated value together with the attribute information and the non-invasive biological information of the user. Note that these pieces of data may be displayed on the terminal device 10 possessed by the user.

<Device hardware configuration>

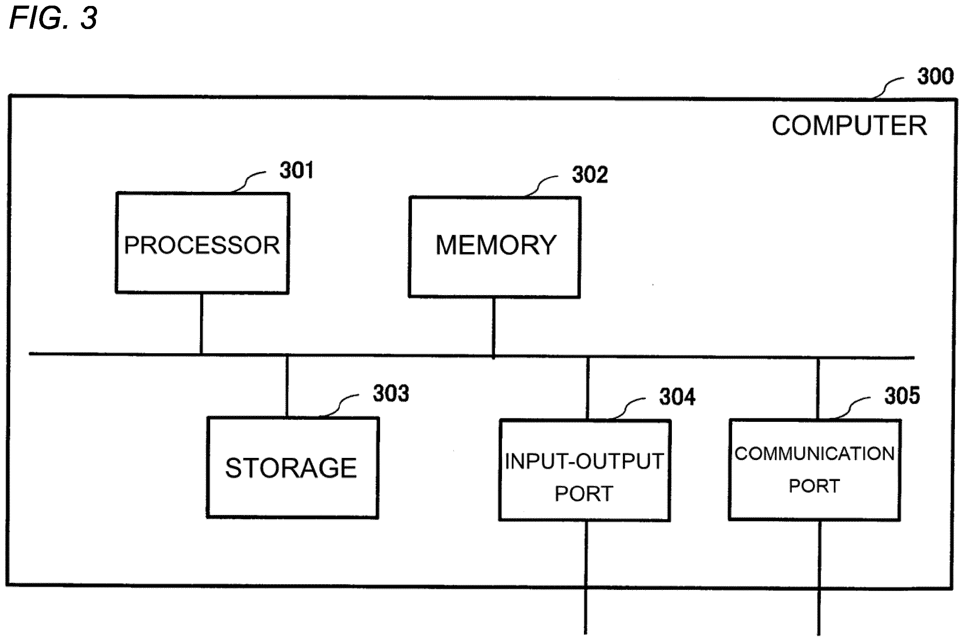

Figure 3 is a hardware configuration diagram of the yGT estimation device 30. As illustrated in Figure 3, the yGT estimation device 30 is configured as a computer 300 including one or a plurality of processors 301, a memory 302, a storage 303, an input-output port 304, and a communication port 305. Each processor 301 performs processing related to yGT estimation according to the present embodiment by executing a computer program. The memory 302 temporarily stores a computer program and a calculation result of the computer program. The storage 303 stores a computer program configured to execute processing by the yGT estimation device 30. The storage 303 may be any computer-readable storage and may be, for example, various kinds of recording media such as a magnetic disk, an optical disk, a random-access memory, a flash memory, and a read-only memory. The input-output port 304 performs inputting of information from the terminal device 10 and the biological information measurement device 20 and outputting of a yGT estimated value to the display device 39. The communication port 305 transmits and receives data to and from a non-illustrated information terminal such as another computer. Communication may be performed by wireless and wired communication methods. Note that the yGT estimation device 30 may be implemented by a commercially available desktop PC or notebook PC, and a time taken for calculation of a yGT estimated value using an estimation model is several seconds.

Note that the first acquisition unit 31, the second acquisition unit 32, the learning processing unit 35, the estimation processing unit 37, and the like described above function at the processors 301 of the yGT estimation device 30 when operating.

It is possible to perform yGT estimation without performing blood examination, by generating a yGT level estimation model by machine learning based on non-invasive biological data including the body mass index (BMI), the blood pressure, the pulse wave data, the electrocardiogram data, the biological impedance, and the like.

As described below, it is also possible to determine whether the yGT is normal even when the number of pieces of data included in the non-invasive biological data is limited.

<Generation of $\gamma$GT estimation model by machine learning>

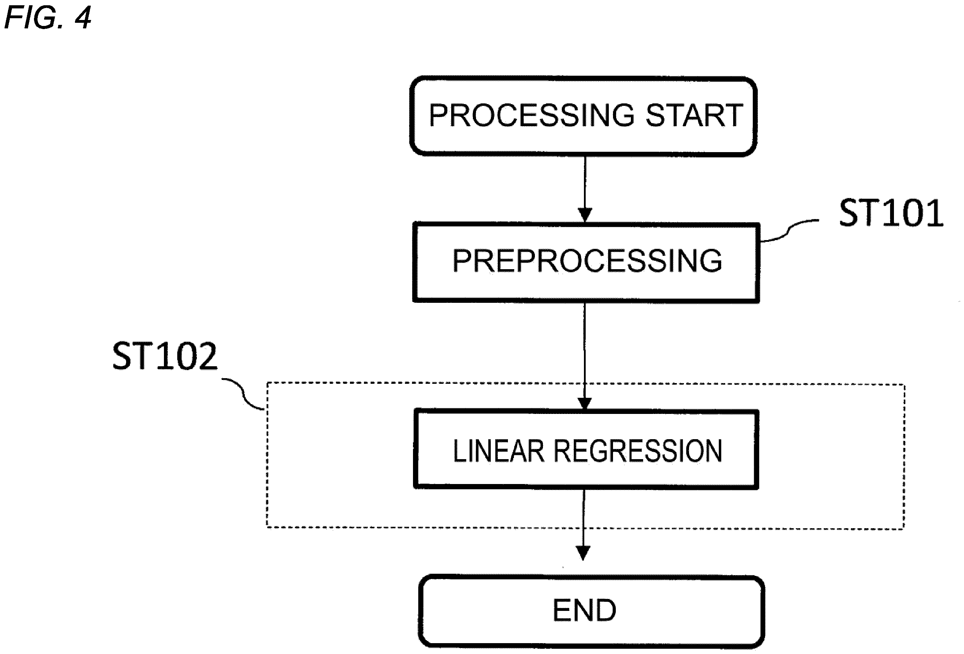

Figure 4 is a flowchart illustrating an execution procedure of generation of a yGT estimation model by machine learning.

At step ST101, the learning processing unit 35 performs preprocessing of input data. Specifically, the learning processing unit 35 converts attribute information of sex of each subject into a One-hot vector. In addition, the learning processing unit 35 normalizes attribute information other than sex, the non-invasive biological information of each subject, and yGT sample examination information obtained through a blood examination through yeo-johnson transformation, boc-box transformation, and the like.

At step ST102, the learning processing unit 35 performs machine learning by the linear regression. For example, linear regression provided in a Python open-source machine learning library Scikit-learn may be used for the machine learning by the linear regression. The learning processing unit 35 may reduce the number of dimensions by primary component analysis as necessary.

The learning processing unit 35 stores the yGT estimation model generated by the above-described learning processing in the estimation model storage unit 36.

Note that the above-described machine learning algorithm is exemplary and the present invention is not limited thereto.

<Estimation of yGT using yGT estimation model>

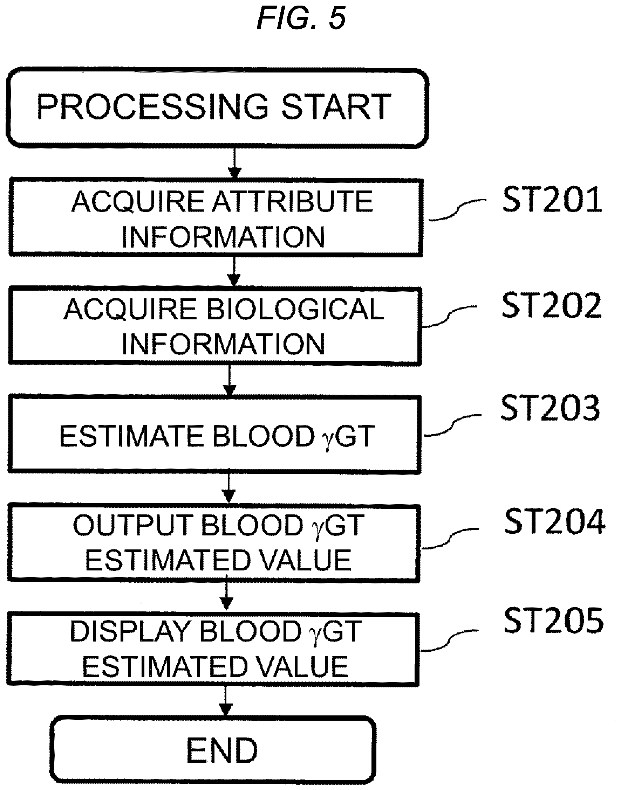

Figure 5 is a flowchart illustrating an execution procedure of yGT estimation processing.

At step ST201, the first acquisition unit 31 of the yGT measurement device 30 acquires the attribute information of the user from the terminal device 10. At step ST202, the second acquisition unit 32 of the yGT measurement device 30 acquires the non-invasive biological information of the user. Then, the attribute information and the non-invasive biological information of the user are stored in the user data storage unit 33. Then at step ST203, the estimation processing unit 37 calculates a yGT estimated value by using the yGT estimation model stored in the estimation model storage unit 36.

At step ST204, the calculated yGT estimated value is output to and stored in the estimation data storage unit 38, and at step ST205, the yGT estimated value is displayed on an external terminal such as the display device 39.

<Examples (yGT estimation)>

Examples of the present application invention will be described below. However, the present invention is not limited to the examples below.

The attribute information includes any one or a combination of ID, full name, age, and sex, and the non-invasive biological information includes any one or a combination of the BMI, the blood pressure, the pulse wave data, the electrocardiogram data, the biological impedance, and the oxygen saturation (SpO2). Height and weight as calculation references of the BMI were measured by a height meter and a weight meter, respectively, and the blood pressure was measured by a blood pressure meter. The pulse wave data, the electrocardiogram data, the biological impedance, and the oxygen saturation (SpO2) were measured by ES-TECK BC-3 (Ryobi Systems Co., Ltd.). Note that a pulse wave meter, an electrocardiogram meter, an impedance measurement device, and a pulse oximeter that are commercially available may be used in combination in place of ES-TECK BC-3. The above-described non-invasive biological information may be acquired by using a predetermined wearable terminal.

The biological impedance (conductance) was measured by generating small current flow between two electrodes among electrodes at the six sites of the legs, the hands, and the right and left foreheads. Voltage and current were 1.28 V and 200 $\mu$A, respectively, and the conductance was measured for 32 milliseconds per second. Current was caused to flow between two of the electrodes at the six sites to measure (1) anode/cathode conductance ($\mu$S), (2) cathode/anode conductance ($\mu$S), (3) the difference (delta SCRA-SCRC) between the conductance measured in (1) above and the conductance measured in (2) above, and (4) electric conductivity ($\mu$S/m).

In addition, the muscle mass, the body fat amount, the total moisture content, the phase angle, and the resistance value were measured, and the dielectric constant ($\mu$Si) was measured for cases of conduction between the right hand and the left hand and between the right forehead and the left forehead.

The pulse wave data, the electrocardiogram data, the biological impedance, and the oxygen saturation (SpO2) were measured for two minutes by using ES-TECK BC-3 for each subject. At the measurement, a device having functions of an electrocardiogram, a pulse wave meter, and a pulse oximeter was mounted on the left hand forefinger of each subject, two electrodes were mounted on the forehead, and the hands and feet were placed on electrode plates while the subject was seated on a chair.

<Learning model 1>

With a learning model 1, machine learning by a linear regression was performed as illustrated in Figure 4.

In this case, data described below was selected and used as the attribute information and the non-invasive biological data. (A) the attribute information: sex and age, (B) the non-invasive biological data: the weight, the circulating blood amount, the blood pressure, the average arterial blood pressure, the diastolic blood pressure, pulse wave data, the elasticity index, the ejection fraction, the biological impedance, the lean body weight (kg), the body fat amount (kg), the muscle mass (%), the total moisture content (kg), the cardiac output, 1 forehead left-side - 2 left hand/SCRA, ESG 2+4+15+17 ($\mu$S/m), ESG 6+8+19+21 ($\mu$S/m), EGS 9+10 ($\mu$S/m), the forehead-path electric conductivity, the hand-to-hand electric conductivity, electrocardiogram data, the heart rate, the standard deviation of the RR interval, and LF/HF, where ESG 9+10 is the average of impedance values measured at the sites illustrated in Figure 2. The unit [$\mu$S/m] is the unit of a measured average, and the unit [%] is a value obtained by scaling the measured average into the range of normally measured values.

In addition, the non-invasive biological data includes the single-output amount obtained by dividing the cardiac output by the heart rate, and the oxygen transport amount calculated based on SpO2 and the cardiac output.

<Example 1>

In Example 1, the yGT estimation model was generated through machine learning of the above-described learning model 1 by using (1) the attribute information, (2) the non-invasive biological information measured by a height-weight meter, a blood pressure meter, and ES-TECK BC-3, and (3) a training data set of yGT obtained through a blood examination performed on the same day as the non-invasive biological information measurement for a total of 712 subjects. Note that the logarithm of the yGT measured value obtained through a blood examination was used in the machine learning.

Then, the estimation accuracy of the yGT estimation model was determined by calculating the coefficient of correlation with the logarithm of the yGT measured value obtained through a blood examination.

As a result, the average, the standard deviation, and the standard error of error between the yGT estimated value and the actual yGT were 5.64, 46.49, and 3.48, respectively. The coefficient of correlation between the logarithm of the yGT actual value and the logarithm of the predicted yGT was 0.61. A test of no correlation obtained a p value less than 0.001, and thus correlation exists between the yGT level estimated by the estimation model and the yGT level obtained through a blood examination.

As described above, it is possible to estimate yGT without performing a blood examination, by generating a yGT estimation model by machine learning based on the non-invasive biological data including the body mass index (BMI), the blood pressure, the pulse wave data, the electrocardiogram data, and the biological impedance.

As described below, it is possible to determine "yGT risk", in other words, whether the yGT level is normal even when the number of pieces of data included in the non-invasive biological data is limited.

<Generation of yGT risk estimation model by machine learning>

According to the present invention, the yGT risk estimated value may be calculated in place of the yGT estimated value.

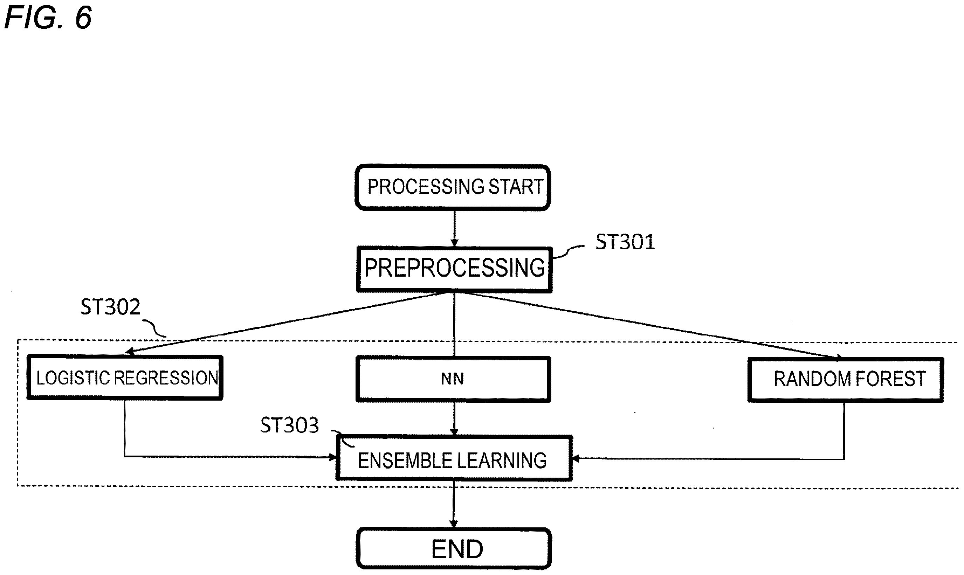

Figure 6 is a flowchart illustrating an execution procedure of generation of a yGT risk estimation model by machine learning.

At step ST301, the learning processing unit 35 performs preprocessing of input data. Specifically, for the measured value of yGT obtained through a blood examination, the learning processing unit 35 converts the yGT equal to or lower than 50 U/L into "0" (no risk is present) and the yGT equal to or higher than 51 U/L into "1" (risk is present). When the number of subjects classified as "0" and the number of subjects classified as "1" are deviated and unbalanced, the learning processing unit 35 may apply SMOTE (Chawla, NV et al. 2002) to learning data to artificially generate training samples. Specifically, a label (for example, above-described "0" or "1") indicating existence of the yGT risk may be provided to each input data (training data set) based on the measured value of yGT obtained through a blood examination. Then, when the difference between the number of labels with the yGT risk (label "1") and the number of labels without the yGT risk (label "0") is equal to or larger than a predetermined value, the number of pieces of sample data in the training data set may be increased (generated) to reduce the difference.

At step ST302, the learning processing unit 35 performs machine learning by a neural network, random forest, and logistic regression illustrated in Figure 6 in parallel.

At step ST303, the learning processing unit 35 further performs ensemble learning (voting) with results of the machine learning by the logistic regression, the neural network (NN), and the random forest.

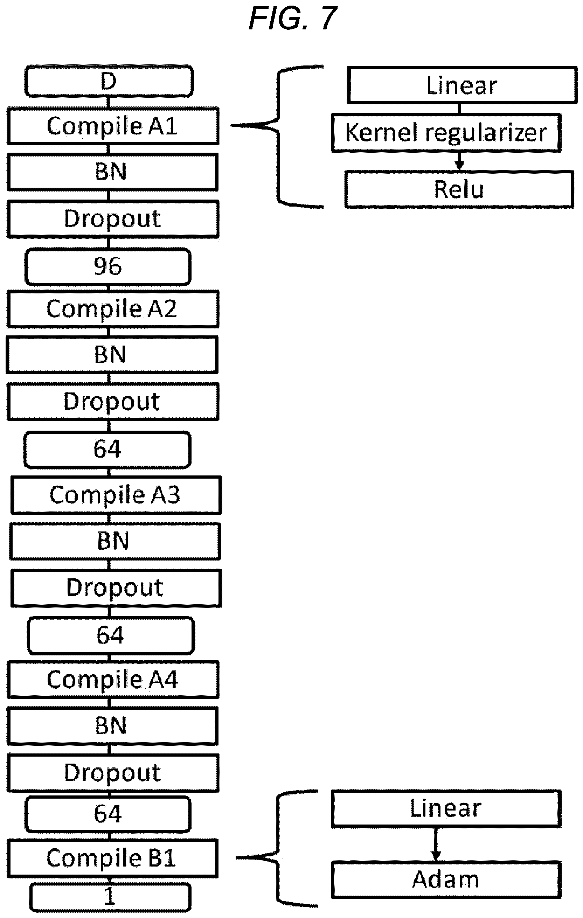

Figure 7 illustrates the structure of a neural network (NN) used as the yGT risk estimation model. "D" represents the number of examination items. A rectangle represents a layer group that performs data conversion, and a rounded rectangle represents input-output data. The structure of the neural network (NN) converts data in the order of D dimensions, 96 dimensions, 64 dimensions, 64 dimensions, 64 dimensions, and one dimension through five layer groups (Compile A1, Compile A2, Compile A3, Compile A4, and Compile B1).

Compile A1, A2, A3, and A4 each include a full connection layer "Linear" that performs full connection processing and a "ReLU" layer that performs ReLU processing, and the layer group Compile B1 includes a full connection layer "Linear" that performs full connection processing and an "Adam" layer that performs optimization processing. An input unit of the full connection layer of the layer group Compile A1 corresponds to an input layer, an output unit of the layer group Compile B1 corresponds to an output layer, and each unit therebetween corresponds to an intermediate layer (hidden layer). The intermediate layer includes a Dropout layer that limits some input values to zero and prevents overfitting and a batch normalization (BN) layer that performs normalization for each minibatch at learning.

For example, Logistic Regression provided in a Python open-source machine learning library Scikit-learn may be used for the machine learning by logistic regression. The number of dimensions may be reduced by primary component analysis as necessary. The yGT risk obtained through a blood examination was compared with the yGT risk estimated by machine learning, and parameters (C, regularization method, max_iter, and solber) of Logistic Regression were adjusted to obtain the maximum fl score. The parameter "C" is a tradeoff parameter that determines the strength of regularization, and the strength of regularization is weaker as the value of the parameter is larger. The parameter "regularization method" means L1 regularization or L2 regularization to be selected. The parameter "max_iter" is the maximum number of iterations of learning. The parameter "solber" selects a convergence method (for example, the L-BFGS method, the Newton CG method, liblinear, sag, or saga) that minimizes the cross-entropy loss. Note that the liblinear method was selected in Examples 2 and 3 below.

For example, Random Forest Classifier provided in a Python open-source machine learning library Scikit-learn may be used for the machine learning by random forest. The yGT risk obtained through a blood examination was compared with the yGT risk estimated by machine learning, and parameters (max_depth, max_features, and n_estimators) of Random Forest Classifier were adjusted to obtain the maximum f1 score. The parameter "max_depth" is the depth of a decision tree, and the parameter "max_features" designates the randomness of each tree. The parameter "n_estimators" is the number of decision trees to be established. However, max_depth was None or 1 to 7, subsample was 0.3 to 0.7, max_features was optimally selected from among "auto", "sqrt", and "log", and n_estimators was adjusted in the range of 10 to 1000.

The learning processing unit 35 stores the yGT risk estimation model generated by the above-described learning processing in the estimation model storage unit 36.

Note that the above-described machine learning algorithm is exemplary and the present invention is not limited thereto.

<Estimation of yGT risk using yGT risk estimation model>

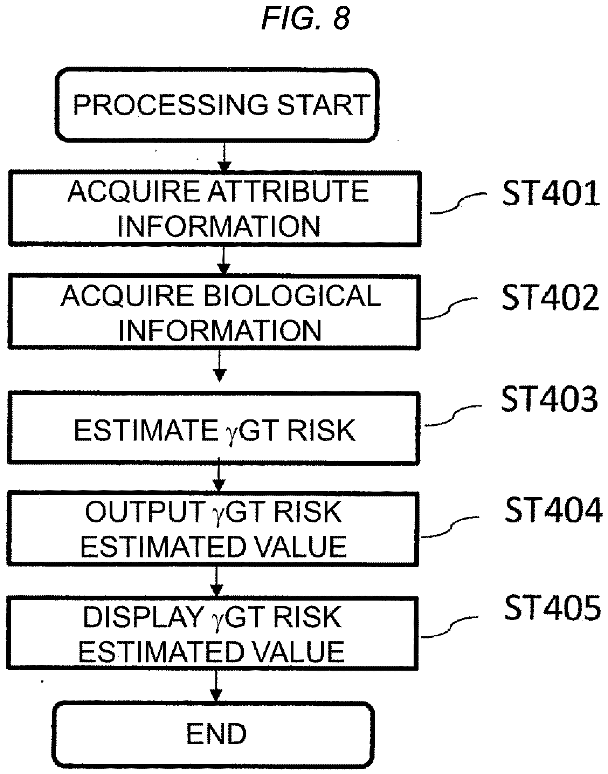

As illustrated in Figure 8, at step ST401, the first acquisition unit 31 of the yGT risk estimation device 30 acquires the attribute information of the user from the terminal device 10. At step ST402, the second acquisition unit 32 of the yGT risk estimation device 30 acquires the non-invasive biological information of the user. Then, the attribute information and the non-invasive biological information of the user are stored in the user data storage unit 33. Then at step ST403, the estimation processing unit 37 calculates probability that the user belongs to class 0 (no risk is present) or class 1 (risk is present), in other words, a yGT risk estimated value by using the yGT risk estimation model stored in the estimation model storage unit 36. At step ST404, the calculated yGT risk estimated value is stored in the estimation data storage unit 38. At step ST405, the yGT risk estimated value is output to an external terminal such as the display device 39 and displayed.

<Examples (yGT risk estimation)>

Examples of γGT risk estimation will be described below. However, aspects of the yGT risk estimation in the present invention are not limited to the examples below.

The attribute information includes ID, full name, age, and sex, and the non-invasive biological information includes the BMI, the blood pressure, the pulse wave data, the electrocardiogram data, the biological impedance, and the oxygen saturation (SpO2). Height and weight as calculation references of the BMI were measured by a height meter and a weight meter, respectively, and the blood pressure was measured by a blood pressure meter. The pulse wave data, the electrocardiogram data, the biological impedance, and the oxygen saturation (SpO2) were measured by ES-TECK BC-3 (Ryobi Systems Co., Ltd.). Note that a pulse wave meter, an electrocardiogram meter, an impedance measurement device, and a pulse oximeter that are commercially available may be used in combination in place of ES-TECK BC-3. The above-described non-invasive biological information may be acquired by using a predetermined wearable terminal.

The biological impedance (conductance) was measured by generating small current flow between two electrodes among electrodes at the six sites of the feet, the hands, and the right and left foreheads. Voltage and current were 1.28 V and 200 μA, respectively, and the conductance was measured for 32 milliseconds per second. Current was caused to flow between two of the electrodes at the six sites to measure (1) anode/cathode conductance (μS), (2) cathode/anode conductance (μS), (3) the difference (delta SCRA-SCRC) between the conductance measured in (1) above and the conductance measured in (2) above, and (4) electric conductivity ($\mu$S/m).

In addition, the muscle mass, the body fat amount, the total moisture content, the phase angle, and the resistance value were measured, and the dielectric constant ($\mu$Si) was measured for cases of conduction between the right hand and the left hand and between the right forehead and the left forehead.

The pulse wave data, the electrocardiogram data, the biological impedance, and the oxygen saturation (SpO2) were measured for two minutes by using ES-TECK BC-3 for each subject. At the measurement, a device having functions of an electrocardiogram, a pulse wave meter, and a pulse oximeter was mounted on the left hand forefinger of each subject, two electrodes were mounted on the forehead, and the hands and feet were placed on electrode plates while the subject was seated on a chair.

<Leaming model 2>

With a learning model 2, as illustrated in Figure 6, machine learning by the logistic regression and the random forest was performed in parallel with machine learning by the neural network, and ensemble learning by voting was performed with results of the learning to produce the yGT risk estimation model. In this case, data described below was selected and used as the non-invasive biological data. (A) the attribute information: age, (B) the non-invasive biological data: the weight, the BMI, the circulating blood amount, the blood pressure, the systolic blood pressure, the diastolic blood pressure, pulse wave data, the elasticity index, electrocardiogram data, the breathing rate, the heart rate, the MxDMn ratio, the biological impedance, the total moisture content (kg), ESG 2+4+15+17 ($\mu$S/m), and the hand-to-hand dielectric constant.

<Leaming model 3>

With a learning model 3, as illustrated in Figure 6, machine learning by the logistic regression was performed. In this case, data described below was selected and used as the non-invasive biological data. (A) the attribute information: sex and age, (B) the non-invasive biological data: the BMI, the blood pressure, the systolic blood pressure, the pulse pressure, pulse wave data, the peripheral vascular resistance, pulse, electrocardiogram data, the breathing rate, the heart rate, the biological impedance, the muscle mass (%), 15 right hand -16 forehead left-side/SCR A, ESG 2+4+15+17 ($\mu$S/m), ESG 6+8+19+21 ($\mu$S/m), EGS 9+10 ($\mu$S/m), ESG 9+10 (%), and R ($\Omega$).

The non-invasive biological data also includes pulse pressure/pulse data, and the oxygen transport amount calculated based on SpO2 and the cardiac output.

<Example 2>

In Example 2, the yGT risk estimation model was generated through machine learning of the above-described learning model 2 by using (1) the attribute information, (2) the non-invasive biological information measured by a height-weight meter, a blood pressure meter, and ES-TECK BC-3, and (3) a training data set of yGT obtained through a blood examination performed on the same day as the non-invasive biological information measurement for 321 subjects.

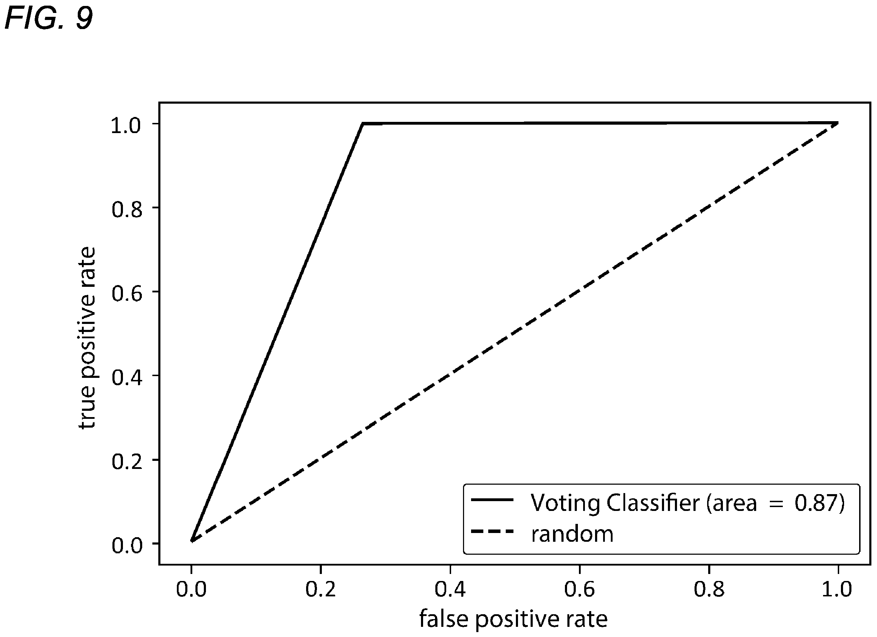

Then, an estimation result was evaluated by using an ROC_AUC curve for the estimation accuracy of the yGT risk estimation model. As a result, ROC_AUC was 0.87, which exceeds 0.8 indicating extremely favorable classification. The ROC_AUC curve of the estimation result in Example 2 is illustrated in Figure 9.

<Example 3>

In Example 3, the yGT risk estimation model was generated through machine learning of the above-described learning model 3 by using (1) attribute information, (2) the non-invasive biological information measured by a height-weight meter, a blood pressure meter, and ES-TECK BC-3, and (3) a training data set of yGT obtained through a blood examination performed on the same day as the non-invasive biological information measurement for a total of 712 subjects.

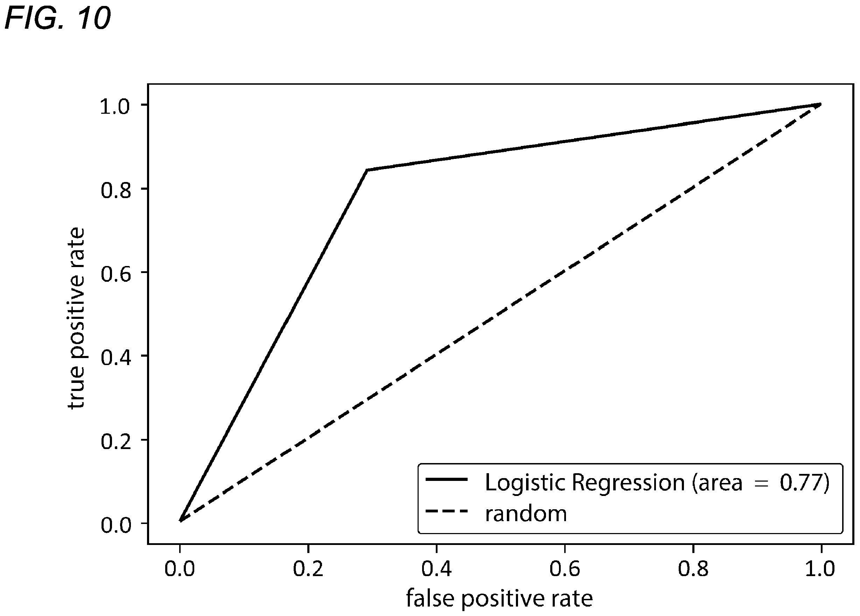

Then, an estimation result was evaluated by using an ROC_AUC curve for the estimation accuracy of the yGT risk estimation model. As a result, ROC_AUC was 0.77, which exceeds 0.7 indicating favorable classification. The ROC_AUC curve of the estimation result in Example 3 is illustrated in Figure 10.

(Modifications)

In the above-described embodiment, the yGT risk is estimated by using the learning model 2 or the learning model 3, but the yGT risk may be estimated by using a plurality of learning models.

Accordingly, the yGT risk can be estimated at higher accuracy than when the yGT risk is estimated by using one learning model.

In the above-described embodiment and examples, the estimation accuracy improves in some cases when the BMI is used for a learning model data set, and thus a functional component configured to estimate the BMI may be provided in a yGT risk estimation device.

The BMI is typically not acquired by a wearable terminal nor the like but is calculated based on a height and a weight input by the user. However, when the BMI is estimated, the above-described yGT risk can be acquired by only acquiring biological information, which improves convenience for the user.

The BMI estimation is not limited to a particular method, but for example, it is known that the BMI is correlated with inclination of the abdominal region (predetermined position) of the user. Thus, for example, a predetermined acceleration sensor may be provided on the abdominal region of the user (or a wrist-band wearable terminal or the like including an acceleration sensor may be placed on the abdominal region), and the BMI may be estimated by calculating the inclination of the abdominal region based on data output from the acceleration sensor.

Similarly to the above description, the pulse wave data or the oxygen saturation may be estimated by using a wrist-band wearable terminal or the like including a pulse wave sensor or a blood oxygen level sensor.

Similarly to the above description, the blood pressure may be estimated by using a wrist-band wearable terminal or the like. Since it is known that the blood pressure is correlated with the speed of a pulse wave transferred through an artery by heartbeat, the blood pressure may be estimated by using a predetermined sensor configured to measure the speed of a pulse wave transferred through an artery by heartbeat.

Similarly to the above description, the electrocardiogram data may be estimated by using a wrist-band wearable terminal or the like. For example, the electrocardiogram data may be estimated based on data obtained from an electrode provided on a surface on a side opposite a display surface of a wrist-band wearable terminal and an electrode provided on the display surface side. Specifically, the electrocardiogram data may be estimated based on data obtained when the wrist of a hand (for example, the left hand) on which the wrist-band wearable terminal is mounted contacts the electrode provided on the opposite surface and a fingertip of a hand (for example, the right hand) opposite to the mounted hand contacts the electrode provided on the display surface side.

Similarly to the above description, the biological impedance may be estimated by using a wrist-band wearable terminal or the like including various electrodes. For example, the biological impedance may be estimated based on biological information obtained from the thoracic region and a wrist by using a wrist-band wearable terminal or the like.

Note that the above-described estimation target biological information (at least one piece or more of biological information among the BMI, the blood pressure, the pulse wave data, the electrocardiogram data, the biological impedance, and the oxygen saturation) may be estimated by using a classifier generated by using various kinds of machine learning algorithms with, as teacher data, biological information that is acquirable by a wearable terminal and the above-described estimation target biological information.

In this case, the above-described second acquisition unit may acquire the estimated biological information.

(Other)

For example, the series of above-described processing may be executed by hardware or software. In other words, the above-described functional configurations are merely exemplary and not particularly limited. Specifically, it suffices that the functionality of executing the entire series of above-described processing is provided at an information processing system, and which functional blocks are used to achieve the functionality is not particularly limited to the above-described example. Places at which functional blocks exist are not particularly limited to those in Figure 1 but may be optional. For example, functional blocks of a server may be transferred to another terminal or device. Functional blocks of another terminal or device may be transferred to a server or the like. Each functional block may be configured by hardware only, by software only, or by combination thereof.

When the series of processing is executed by software, computer programs constituting the software are installed on a computer or the like from a network or a recording medium. The computer may be a computer incorporated in dedicated hardware. Alternatively, the computer may be a computer capable of executing various functions with various computer programs installed thereon, such as a server or a general-purpose smartphone or personal computer.

A recording medium including such computer programs is not only configured as a non-illustrated removable media distributed separately from the device body to provide the computer programs to a user or the like, but also configured as, for example, a recording medium incorporated in the device body in advance and provided to the user or the like. The computer programs can be distributed through a network, and thus the recording medium may be mounted on or accessible from a computer connected or connectable to the network.

Note that, in the present specification, steps representing the computer programs recorded in the recording medium include not only processing performed in a temporally sequential manner in accordance with an order but also processing not necessarily processed in a temporally sequential manner but executed in parallel or individually. In the present specification, system terms mean those of an overall device including a plurality of devices, a plurality of units, or the like.

Reference Signs List

| | |
|---|---|
| 1 | yGT estimation system |
| 10 | terminal device |
| 20 | biological information measurement device |
| 30 | yGT estimation device |
| 31 | first acquisition unit |
| 32 | second acquisition unit |
| 33 | user data storage unit |
| 34 | training data storage unit |
| 35 | learning processing unit |
| 36 | estimation model storage unit |
| 37 | estimation processing unit |
| 38 | estimation data storage unit |
| 39 | display device |
| 300 | computer |
| 301 | processor |
| 302 | memory |
| 303 | storage |
| 304 | input-output port |
| 305 | communication port |

**Claims**

1. A yGT estimation device comprising:

   an information acquisition unit configured to acquire attribute information and non-invasive biological information of a predetermined user;
   an estimation model storage unit configured to store a yGT estimation model; and
   an estimation processing unit configured to calculate a yGT estimated value of the predetermined user based on the attribute information and/or the non-invasive biological information of the predetermined user by using the yGT estimation model.

2. The yGT estimation device according to claim 1,

   wherein the attribute information includes any one or a combination of age and sex, and
   wherein the non-invasive biological information includes any one or a combination of BMI, blood pressure, pulse wave data, electrocardiogram data, and biological impedance.

3. The yGT estimation device according to claim 1 or 2, further comprising:

   a training data storage unit configured to store a training data set; and
   a learning processing unit configured to generate the yGT estimation model by machine learning based on the training data set.

4. The yGT estimation device according to claim 3, wherein the training data set includes attribute information, non-invasive biological information, and a blood-measured yGT measured value of a subject.

5. The yGT estimation device according to claim 4, wherein the non-invasive biological information further includes oxygen saturation (SpO2).

6. The yGT estimation device according to claim 4 or 5, wherein a coefficient of correlation between a logarithm of the yGT estimated value and a logarithm of the yGT measured value is equal to or larger than 0.6.

**7.** The yGT estimation device according to any one of claims 3 to 6,

wherein the training data set includes non-invasive biological information and a blood-measured yGT measured value of a subject, and
wherein the estimation processing unit calculates a yGT risk estimated value in place of the yGT estimated value.

**8.** The yGT estimation device according to claim 7,

wherein the learning processing unit provides labels indicating existence of the yGT risk to the training data set based on the blood-measured yGT measured value, and
wherein, when a difference between the numbers of pieces of data with the yGT risk and data without the yGT risk among the labels is equal to or larger than a predetermined value, the learning processing unit increases the number of pieces of sample data in the training data set to reduce the difference.

**9.** The yGT estimation device according to claim 7,

wherein the learning processing unit generates a first yGT risk estimation model and a second yGT risk estimation model by machine learning based on each of training data sets of different kinds, and
wherein the estimation processing unit calculates the yGT risk estimated value of the predetermined user by using the first yGT risk estimation model and the second yGT risk estimation model.

**10.** The yGT estimation device according to any one of claims 1 to 8, further comprising a biological information estimation unit configured to estimate at least one piece or more of biological information among BMI, blood pressure, pulse wave data, electrocardiogram data, biological impedance, and oxygen saturation included in the biological information, wherein the information acquisition unit acquires, as biological information of the predetermined user, the biological information estimated by the biological information estimation unit.

**11.** A non-invasive yGT estimation system comprising:

the yGT estimation device according to any one of claims 1 to 10; and
a biological information measurement device configured to measure non-invasive biological information.

**12.** A yGT estimation method comprising:

a step of storing a training data set including attribute information, non-invasive biological information, and a blood-measured yGT measured value of a subject;
a step of generating a yGT estimation model by machine learning based on the training data set; and
a step of calculating a yGT estimated value of a predetermined user based on attribute information and/or non-invasive biological information of the predetermined user by using the yGT estimation model.

**13.** A computer program configured to cause a computer to execute:

a step of storing a training data set including attribute information, non-invasive biological information, and a blood-measured yGT measured value of a subject;
a step of generating a yGT estimation model by machine learning based on the training data set; and
a step of calculating a yGT estimated value of a predetermined user based on attribute information and/or non-invasive biological information of the predetermined user by using the yGT estimation model.

**FIG. 1**

γGT RISK ESTIMATION DEVICE — 30

- TERMINAL DEVICE — 10
- BIOLOGICAL INFORMATION MEASUREMENT DEVICE — 20
- FIRST ACQUISITION UNIT — 31
- SECOND ACQUISITION UNIT — 32
- USER DATA STORAGE UNIT — 33
- TRAINING DATA STORAGE UNIT — 34
- LEARNING PROCESSING UNIT — 35
- ESTIMATION MODEL STORAGE UNIT — 36
- ESTIMATION PROCESSING UNIT — 37
- ESTIMATION DATA STORAGE UNIT — 38
- DISPLAY DEVICE — 39

1

## FIG. 2

| ELECTRODE | DIRECTION | ELECTRODE |
|---|---|---|
| 1  LEFT FOREHEAD | ↔ | 2  LEFT HAND |
| 3  RIGHT FOREHEAD | ↔ | 4  RIGHT HAND |
| 5  LEFT HAND | ↔ | 6  LEFT FOOT |
| 7  RIGHT HAND | ↔ | 8  RIGHT FOOT |
| 9  LEFT FOREHEAD | ↔ | 10  RIGHT FOREHEAD |
| 11  LEFT HAND | ↔ | 12  RIGHT HAND |
| 13  LEFT FOOT | ↔ | 14  RIGHT FOOT |
| 15  RIGHT HAND | ↔ | 16  LEFT FOREHEAD |
| 17  LEFT HAND | ↔ | 18  RIGHT FOREHEAD |
| 19  RIGHT FOOT | ↔ | 20  LEFT HAND |
| 21  LEFT FOOT | ↔ | 22  RIGHT HAND |

*FIG. 3*

EP 4 383 264 A1

*FIG. 4*

```
┌─────────────────────────┐
│    PROCESSING START     │
└─────────────────────────┘
              │
              ▼
┌─────────────────────────┐        ST101
│      PREPROCESSING      │
└─────────────────────────┘
              │
  ST102       ▼
 ┌ ─ ─ ─ ─ ─ ─ ─ ─ ─ ─ ─ ─ ─ ┐
   ┌─────────────────────────┐
 │ │    LINEAR REGRESSION    │ │
   └─────────────────────────┘
 └ ─ ─ ─ ─ ─ ─ ─ ─ ─ ─ ─ ─ ─ ┘
              │
              ▼
┌─────────────────────────┐
│          END            │
└─────────────────────────┘
```

EP 4 383 264 A1

## FIG. 5

PROCESSING START

ACQUIRE ATTRIBUTE INFORMATION — ST201

ACQUIRE BIOLOGICAL INFORMATION — ST202

ESTIMATE BLOOD γGT — ST203

OUTPUT BLOOD γGT ESTIMATED VALUE — ST204

DISPLAY BLOOD γGT ESTIMATED VALUE — ST205

END

*FIG. 6*

## FIG. 7

```
┌─────────────────────┐        ┌──────────────────────┐
│          D          │        │        Linear        │
├─────────────────────┤   ┐    ├──────────────────────┤
│     Compile A1      │   │    │  Kernel regularizer  │
├─────────────────────┤   ┘    ├──────────────────────┤
│         BN          │        │         Relu         │
├─────────────────────┤        └──────────────────────┘
│       Dropout       │
├─────────────────────┤
│         96          │
├─────────────────────┤
│     Compile A2      │
├─────────────────────┤
│         BN          │
├─────────────────────┤
│       Dropout       │
├─────────────────────┤
│         64          │
├─────────────────────┤
│     Compile A3      │
├─────────────────────┤
│         BN          │
├─────────────────────┤
│       Dropout       │
├─────────────────────┤
│         64          │
├─────────────────────┤
│     Compile A4      │
├─────────────────────┤
│         BN          │
├─────────────────────┤
│       Dropout       │
├─────────────────────┤
│         64          │        ┌──────────────────────┐
├─────────────────────┤        │        Linear        │
│     Compile B1      │   ┐    ├──────────────────────┤
├─────────────────────┤   ┘    │         Adam         │
│          1          │        └──────────────────────┘
└─────────────────────┘
```

*FIG. 8*

```
         PROCESSING START

      ACQUIRE ATTRIBUTE          ST401
         INFORMATION

      ACQUIRE BIOLOGICAL         ST402
         INFORMATION

      ESTIMATE γGT RISK          ST403

       OUTPUT γGT RISK           ST404
       ESTIMATED VALUE

       DISPLAY γGT RISK          ST405
       ESTIMATED VALUE

             END
```

FIG. 9

EP 4 383 264 A1

FIG. 10

## INTERNATIONAL SEARCH REPORT

| International application No. |
|---|
| **PCT/JP2022/015097** |

### A. CLASSIFICATION OF SUBJECT MATTER

*G16H 10/40*(2018.01)i; *G16H 50/70*(2018.01)i; *A61B 5/00*(2006.01)i; *A61B 5/02*(2006.01)i; *A61B 5/145*(2006.01)i; *G06Q 10/04*(2012.01)i
FI: G16H10/40; A61B5/145; A61B5/02 D; G06Q10/04; G16H50/70; A61B5/00 G

According to International Patent Classification (IPC) or to both national classification and IPC

### B. FIELDS SEARCHED

Minimum documentation searched (classification system followed by classification symbols)

G16H10/40; G16H50/70; A61B5/00; A61B5/02; A61B5/145; G06Q10/04

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2020-162834 A (TANITA CORPORATION) 08 October 2020 (2020-10-08) paragraphs [0055]-[0110], fig. 1-15 | 1-13 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **06 June 2022** | **14 June 2022** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 **Japan** | |
| | Telephone No. |

Form PCT/ISA/210 (second sheet) (January 2015)

**INTERNATIONAL SEARCH REPORT**
**Information on patent family members**

International application No.

**PCT/JP2022/015097**

| Patent document cited in search report | | | Publication date (day/month/year) | Patent family member(s) | Publication date (day/month/year) |
|---|---|---|---|---|---|
| JP | 2020-162834 | A | 08 October 2020 | WO 2020/203728 A1 paragraphs [0053]-[0108], fig. 1-15 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004226277 A

**Non-patent literature cited in the description**

- Summary of the clinical investigations E. S. Teck Complex. *Psychology Research and Behavior Management 2011,* 20 March 2010, vol. 4, 81-86
- **R. N. CHUA ; Y. W. HAU ; C. M. TIEW ; W. L. HAU.** Investigation of Attention Deficit/Hyperactivity Disorder Assessment Using Electro Interstitial Scan Based on Chronoamperometry Technique. *IEEE Access,* 2019, vol. 7, 144679-144690
- **MAAREK A.** Electro interstitial scan system: assessment of 10 years of research and development. *Med Devices (Auckl),* 2012, vol. 5, 23-30